# EUROPEAN PATENT APPLICATION

(11) **EP 1 844 778 A1**
(43) Date of publication of application: **17.10.2007**
(21) Application number: 06712779.5
(22) Date of filing: 01.02.2006
(51) Int. Cl.: A61K 31/4545, A61K 31/496, A61P 17/00, A61P 17/06, C07D 213/38, C07D 401/06, C07D 401/14

(54) **AGENT FOR PREVENTION/THERAPY OF DISEASE CAUSED BY KERATINOCYTE GROWTH**

(30) Priority: 02.02.2005 JP 2005026178
(71) Applicant: Kowa Company. Ltd., Nagoya-shi, Aichi 460-8625 (JP)
(72) Inventor: EDANO, Toshiyuki, Saitama, 3500034 (JP); TABUNOKI, Yuichiro, Tokyo, 2030051 (JP); KOSHI, Tomoyuki, Saitama, 3530006 (JP)
(74) Representative: Hartz, Nikolai
(86) International application number: PCT/JP2006/301636
(87) International publication number: WO 2006/082834

(57) **Abstract**

The present invention relates to an agent for the prevention/therapy of a disease caused by keratinocyte growth, which comprises, as an active ingredient, a compound represented by General Formula (1): 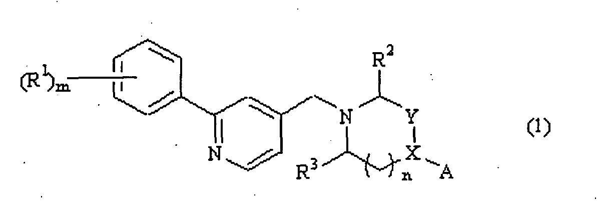 wherein A represents a group selected from groups of the following formulas: 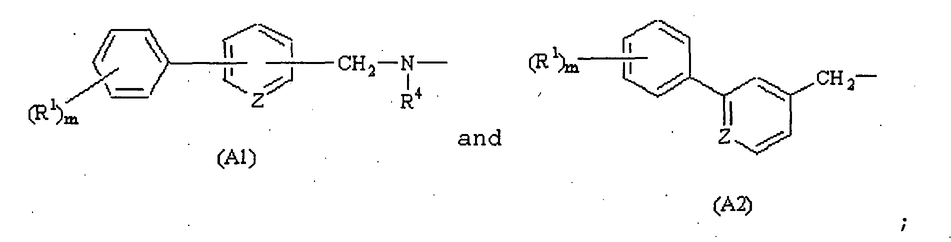 X represents CH or N;
Y represents CH₂ or C=O;
Z represents CH or N;
R¹ represents an alkoxy group;
R² and R³ each independently represent an alkyl group;
R⁴ represents an alkyl group, and a group of the following formula: 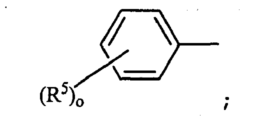 R⁵ each independently represent an alkoxy group or an alkylthio group;
m represents an integer from 1 to 3;
n represents an integer of 1 or 2; and
o represents an integer of 1 or 2,
or a salt thereof, or a solvate of the compound or the salt.

## Description

### Field of the Invention

The present invention relates to an agent for the prevention/therapy and method for the prevention/therapy of a disease caused by keratinocyte growth such as psoriasis.

### Description of the Related Art

Psoriasis is a skin disease which belongs to a class of inflammatorykeratoses, and is a non-infectious disease in which silvery white dry dandruff-like scales are adhered to a red raised rash (erythema), and the scales eventually flake off. Psoriasis appears when corneous keratinocytes excessively outgrow, inflammatory cells accumulate, and excessive neoangiogenesis occurs in the stratum corneum. Psoriasis is clinically classified into psoriasis vulgaris, psoriatic erythrodermia, arthropathic psoriasis, guttate psoriasis, and pustular psoriasis. The cause of onset is still unclear, but it is believed that a change in the keratinocyte's metabolism is the most critical factor for the induction of the disease. Psoriasis is considered to be a disease that is affected by multiple factors such as genetic factors and environmental factors. No fundamental treatment for the disease has been developed, and thus the purpose of its treatment focuses on prolonging the period in which the symptoms are alleviated.

For the therapeutic agents currently used in the clinical field, steroid drugs, etretinate, vitamin preparations such as retinoids, Bonalfa (registered trademark), immunosuppressants such as methotrexate, cyclosporine, may be mentioned. However, these therapeutic agents show unsatisfactory effects or have problems of side effects, and thus none of them is sufficiently satisfactory (Non-patent documents 1 to 3). For example, while steroid drugs exhibit some effects, they are known to cause side effects such as atrophy of skin and capillary dilation. Retinoids (vitamin A) also have side effects such as teratogenesis, mucocutaneous toxicity, arthralgia or myalgia, and hepatic damage (Non-patent document 4). Furthermore, activated vitamin D3 preparations are associated with hypercalcemia or local irritating sensations as side effects (Non-patent document 1 and 3). Cyclosporine exhibit side effects such as a transient increase in blood pressure and renal function impairment (Non-patent document 5).
Recently, biological preparations such as antibodies suppressing the activation of T-cells, fusion proteins, recombinant interleukins have been developed as therapeutic agents for psoriasis, but economic efficiency is an important problem with these preparations (Non-patent documents 6 and 7). Therefore, it is desired to develop a therapeutic agent for psoriasis, which is more useful from the viewpoints of effect, safety and economic efficiency.
Patent document 1 : WO 03/002536
Patent document 2 : WO 03/002554
Patent document 3 : WO 03/020703
Non-patent document 1 : Expert Opin. Investig. Drugs (2000) 9, 79-93
Non-patent document 2 : Expert Opin. Investig. Drugs (2003) 12, 1111-1121
Non-patent document 3 : Lancet (2003) 361, 1197-1204
Non-patent document 4 : Dermatol. Clin. (2004) 22, 467-476
Non-patent document 5 : Br. J. Dermatol. (2004) 150, 1-10
Non-patent document 6 : Expert Opin. Pharmacother. (2003) 4, 1525-1533
Non-patent document 7 : Expert Opin. Pharmacother. (2003) 4, 1887

### Disclosure of the Invention

### Problems to be solved by the Invention

Accordingly, it is an object of the present invention to provide a more useful agent for the prevention/therapy of a disease caused by keratinocyte growth, which is typified by psoriasis, and a method for the prevention/therapy of the disease.

### Means for solving the problems

The inventors of the present invention previously have synthesized a variety of active compounds during a course of research on inhibitory agents for cell adhesion induction, and filed patent applications on such compounds (Patent documents 1 to 3). Surprisingly, they found that a compound represented by the following General Formula (1), which is one of the compounds described above, has an inhibitory effect on keratinocyte growth, and completed the present invention.

Thus, according to an aspect of the present invention, there is provided an agent for the prevention/therapy of a disease caused by keratinocyte growth, the agent comprising, as an active ingredient, a compound represented by General Formula (1):

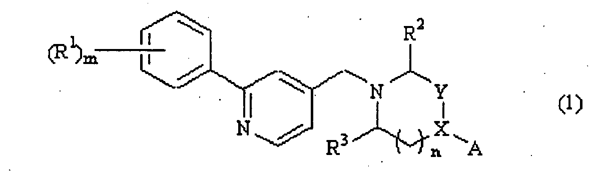

wherein A represents a group selected from a group of the following formulas:

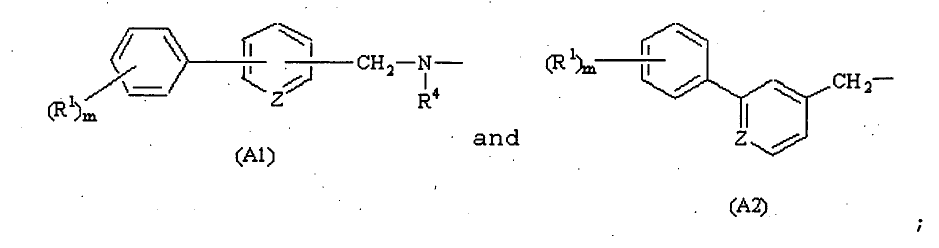

X represents CH or N;
Y represents CH₂ or C=O;
Z represents CH or N;
R¹ represents a C₁-C₆ straight-chained or branched alkoxy group;
R² and R³ each independently represent a C₁-C₆ straight-chained or branched alkyl group;

R⁴ represents a group selected from C₁-C₆ straight-chained or branched alkyl groups and a group of the following formula:

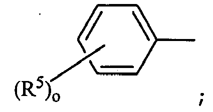

R⁵ each independently represent a C₁-C₆ straight-chained or branched alkoxy group or a C₁-C₆ straight-chained or branched alkylthio group;
m represents an integer from 1 to 3;
n represents an integer of 1 or 2; and
o represents an integer of 1 or 2,
or a salt thereof, or a solvate of the compound or the salt.

According to another aspect of the invention, there is provided a method for the prevention/therapy of a disease caused by keratinocyte growth, the method comprising administering an effective amount of the compound represented by General Formula (1), a salt thereof, or a solvate of the compound or the salt.

According to another aspect of the invention, there is provided use of the compound represented by General Formula (1), a salt thereof, or a solvate of the compound or the salt, for the manufacture of an agent for the prevention/therapy of a disease caused by keratinocyte growth.

### Effect of the Invention

According to embodiments of the present invention, an agent for the prevention/therapy of a disease caused by keratinocyte growth, for example, psoriasis, and a method for the prevention/therapy of the disease can be provided.

### Brief description of the drawings

Fig. 1 is a graph showing the inhibitory effect of a compound according to an embodiment of the present invention on the skin thickening in TPA-stimulated hairless mouse; and
Fig. 2 is a graph showing a comparison of the inhibitory effect on the growth of cultured human epidermal keratinocyte and the VCAM-1 expression inhibitory action of a compound according to an embodiment of the present invention.

### Description of the preferred embodiments

With regard to General Formula (1), the C₁-C₆ straight-chained or branched alkoxy group of R¹ may be exemplified by a methoxy group, an ethoxy group, an n-propoxy group, an isopropoxy group, an n-butoxy group, an isobutoxy group, a sec-butoxy group, a t-butoxy group, a pentyloxy group, or a hexyloxy group. Among these, a methoxy group is preferred.

The C₁-C₆ straight-chained or branched alkyl, group of R² and R³ may be exemplified by a methyl group, an ethyl group, an n-propyl group, an isopropyl group, an n-butyl group, an isobutyl group, a sec-butyl group, a t-butyl group, a pentyl group, or a hexyl group. Among these, a methyl group and an isopropyl group are preferred.

The C₁-C₆ straight-chained or branched alkyl group of R⁴ may be exemplified by the same substituents as those described above. Among them, a methyl group is preferred.
The C₁-C₆ straight-chained or branched alkoxy group of R⁵ maybe exemplified by the same substituents as those described above. Among them, a methoxy group is preferred.
The C₁-C₆ straight-chained or branched alkylthio group of R⁵ may be exemplified by a methylthio group, an ethylthio group, an n-propylthio group, an isopropylthio group, an n-butylthio group, an isobutylthio group, a sec-butylthio group, a t-butylthio group, a pentylthio group, or a hexylthio group. Among these, a methylthio group is preferred.

m represents an integer from 1 to 3, and 3 is particularly preferred.
n represents an integer of 1 or 2, and 1 is particularly preferred.
o represents an integer of 1 or 2.

It is preferable that in the General Formula (1), (R¹)ₘ- is substituted with methoxy groups on the 3-, 4- and 5-positions on the benzene ring. Also, between the Formulas (A1) and (A2), Formula (A1) is particularly preferred. In the General Formula (1), X is preferably CH, Y is preferably CH₂, and n is preferably 1. In this case, the ring containing X and Y is a piperidine ring.

With regard to the General Formula (1), a compound represented by the following Formula (1a) is more preferred:

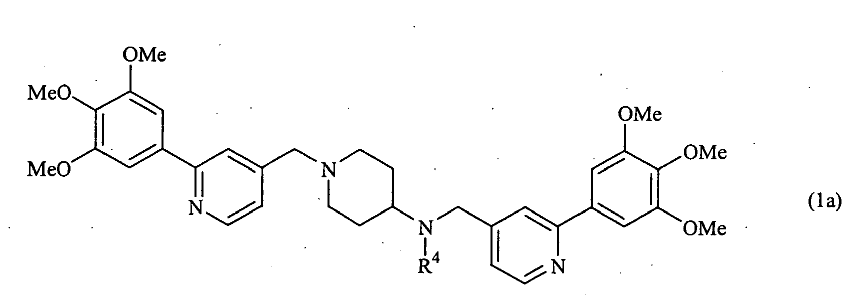

wherein R⁴ has the same meaning as defined above. Here, R⁴ is particularlypreferablyamethyl group, amethylthiophenyl group, a methoxyphenyl group, or a dimethoxyphenyl group.

With regard to the General Formula (1), a compound selected from compounds of the following formulas:

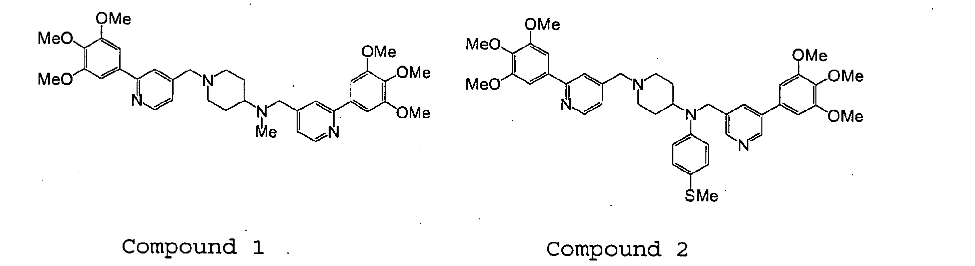

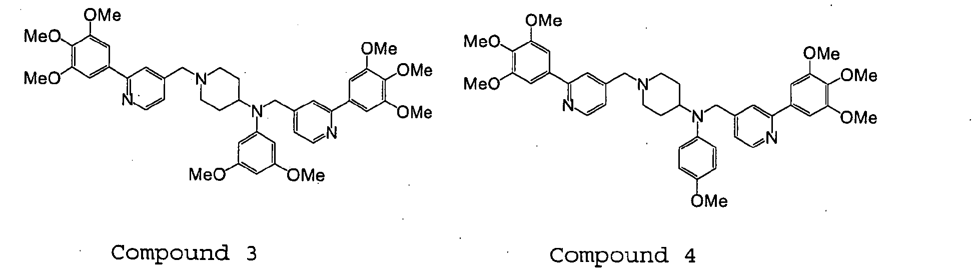

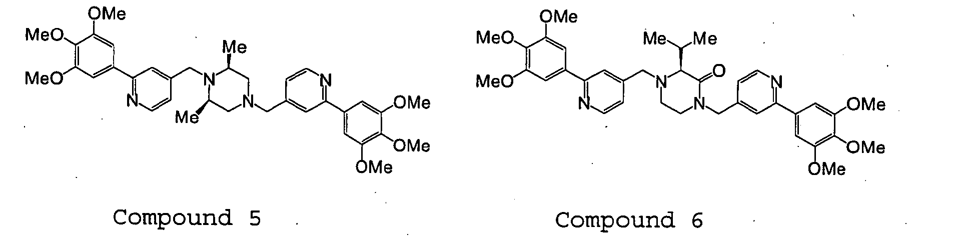

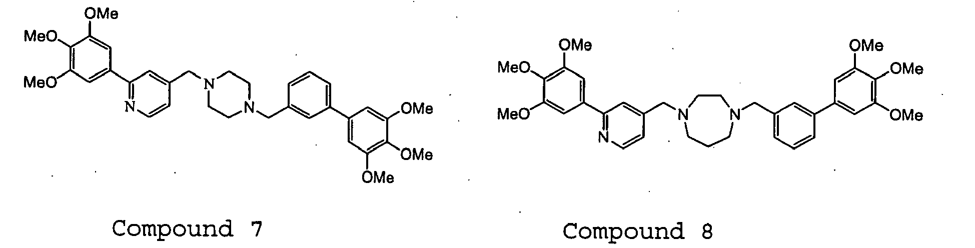

or a salt thereof, or a solvate of the compound or the salt is preferred. Furthermore, the Compounds 1 to 4 are particularly preferred.

The acid addition salt of the above-described compound is not particularly limited as long as the salt is pharmaceutically acceptable, but examples thereof include acid addition salts of mineral acids such as hydrochloride salt, hydrobromide salt, hydroiodide salt, sulfate, phosphate and nitrate; and acid addition salts of organic acids such as benzoate, methanesulfonate, ethanesulfonate, benzenesulfonate, p-toluenesulfonate, oxalate, maleate, fumarate, tartrate, citrate and acetate.
The compound of General Formula (1) may exist in the form of a solvate, which is typified by a hydrate, and the solvates are also included in the present invention.

The compound of General Formula (1) can be prepared by a method described in the pamphlet of WO 03/002532, WO 03/002536, WO 03/002540, WO 03/002554 or WO 03/020703, or a similar method.

Since the compoundof General Formula (1) strongly inhibits keratinocyte growth, as will be described later in Examples, the compound is useful as a drug for the prevention/therapy of a disease caused by keratinocyte growth, for example, psoriasis. Psoriasis may be exemplified by psoriasis vulgaris, psoriatic erythrodermia, arthropathic psoriasis, guttate psoriasis, pustular psoriasis, or the like.
Furthermore, the inventors evaluated the correlation between the keratinocyte growth inhibitory action and a cell adhesion molecule induction inhibitory action (VCAM-1 expression inhibitory activity), but there was no correlation therebetween. Accordingly, it is conceived that the keratinocyte growth inhibitory action of the compound of the present invention is irrelevant to the cell adhesion molecule induction inhibitory action.

The agent for the prevention/therapy of the present invention contains, as an active ingredient, the compound of General Formula (1), a salt thereof, or a solvate of the compound or the salt, and is prepared in the formof a general pharmaceutical preparation. Such preparation is prepared using a conventionally used diluent or excipient such as a filler, a bulking agent, a binding agent, a moisturizing agent, a disintegrant, a surfactant, a gliding agent or the like. For this pharmaceutical preparation, various dosage forms can be selected in accordance with the purpose of therapy, and representative forms include tablet, pill, powder, solution, suspension, emulsion, granule, capsule, suppository, injection (solution, suspension, etc.), spray preparations such as inhalant, external aerosol, and external preparations such as liquid coating preparation, lotion, liniment, ointment, cream, PAP preparation, while an external preparation is preferred.

When forming into a form of a tablet, a wide range of materials that are conventionally known in the pertinent art as carrier can be used, and examples thereof include excipients suchas lactose, sucrose, sodium chloride, glucose, urea, starch, calcium carbonate, kaolin, crystalline cellulose, silicic acid; binding agents such as water, ethanol, propanol, simple syrup, glucose solution, starch solution, gelatin solution, carboxymethylcellulose, ceramics, methylcellulose, potassium phosphate, polyvinylpyrrolidone; disintegrants such as dry starch, sodium alginate, powdered agar, powdered laminaran, sodium hydrogen carbonate, calcium carbonate, polyoxylethylene sorbitan fatty acid esters, sodium lauryl sulfate, stearic acid monoglyceride, starch, lactose; disintegration suppressants such as sucrose, stearin, cacao butter, hydrogenated oils; absorption promoters such as quaternary ammonium bases, sodium lauryl sulfate; moisturizers such as glycerin, starch; adsorbents such as starch, lactose, kaolin, bentonite, colloidal silicic acid; gliding agents such as purified talc, stearic acid salts, powdered boric acid, polyethylene glycol. Furthermore, if necessary, the tablet may be prepared into a tablet provided with a conventional coating such as, for example, a sugar coated tablet, a gelatin coated tablet, an enteric coated tablet, a film coated tablet, a dual tablet, or a multilayered tablet.

When molding into a form of a pill, a wide range of materials that are conventionally known in the pertinent art as carrier can be used, and examples thereof include excipients such as glucose, lactose, starch, cacao butter, hydrogenated plant oils, kaolin, talc; binding agents such as powdered gum arabic, powdered tragacanth, gelatin, ethanol; disintegrants such as laminaran, agar; and the like. When forming into a form of a suppository, a wide range of materials that are conventionally known in the pertinent art as carrier can be used, and examples thereof include polyethylene glycol, cacao butter, higher alcohols, esters of higher alcohols, gelatin, semisynthetic glycerides and the like.

In the case of preparing into an injectable preparation, it is preferable that the preparation is prepared into a form of a solution, an emulsion or a suspension, and then usually sterilized as well as made isotonic with the blood. When forming into a form of these solution, emulsion and suspension, all of those conventionally used in the pertinent art as a diluent can be used, and examples thereof include water, ethyl alcohol, propylene glycol, ethoxylated isostearyl alcohol, polyoxylated isostearyl alcohol, polyoxyethylene sorbitan fatty acid esters and the like. In addition, to make the preparation isotonic with the blood, common salt, glucose or glycerin is added in an amount sufficient for preparing an isotonic solution. Furthermore, a conventional dissolution aid, a buffering agent, a soothing agent, and further, if necessary, a colorant, a preservative, a perfume, a flavoring agent, a sweetening agent or another drug may also be added to the agent for therapy.

In the case of forming into a form of a spray preparation, a wide range of materials that are conventionally known in the pertinent art as dispersant and propellant can be used, and examples of the dispersant include lecithins such as soybean lecithin, egg yolk lecithin; fatty acids such as oleic acid, linolic acid, linolenic acid; sorbitans such as sorbitan trioleate, sorbitan monooleate; and the like. Examples of the propellant include conventional non-flammable liquefied gases such as Freon 11, Freon 12, Freon 114.

In the case of using the preparation as an ointment, the preparation is prepared using lipophilic base materials, and the lipophilic base materials include the following, namely, hydrocarbons such as liquid paraffin, mineral oil, white petrolatum, yellow petrolatum, petrolatum jelly, paraffin (hard paraffin, paraffin waxes), microcrystalline waxes, polyethylene, squalane, squalene; silicones such as dimethylpolysiloxane, polydimethylsiloxane; higher alcohols such as myristyl alcohol, lauryl alcohol, cetyl alcohol, stearyl alcohol, cetostearyl alcohol, oleyl alcohol, anethole, citronellol, eugenol; sterols or sterol esters such as lanolin, liquid lanolin, lanolin wax, isopropyl lanolin, acetylated lanolin, lanolin alcohol, cholesterol; higher fatty acids such as lauric acid, myristic acid, palmitic acid, stearic acid, oleic acid; esters such as carnauba wax, beeswax, whale wax, polyethylene glycol esters, ethylene glycol esters, glycerin monoesters, sorbitol esters, sorbitan esters, isopropyl myristate, isopropyl palmitate, isopropyl adipate; plant oils such as almond oil, corn oil, cotton seed oil, olive oil, soybean oil, peanut oil, palm oil, fractionated palm oil, sesame oil; and the like. These base materials may be used individually, or may be used as a mixture of two or more species. The therapeutic agent for psoriasis of the present inventionmaybe appropriately mixed, if necessary, with generally used additives such as a metal soap, an animal or plant extract, a medicinal preparation such as a vitamin preparation, a hormone preparation, an amino acid or the like, a surfactant, a colorant, a dye, a pigment, a fragrance, an ultraviolet absorbent, a moisturizing agent, a thickening agent, an antioxidant, a preservative, an absorption promoter, a sequestering agent, a pH adjusting agent.

The amount of the compound of General Formula (1), a salt thereof or a solvate of the compound or the salt, which should be contained in the agent for prevention/therapy of the present invention, is not particularly limited and may be selected from a wide range. However, in the case of an oral preparation, an injectable preparation, a spray preparation or the like, the amount is usually 0.01 to 70% by weight, and preferably 1 to 50% by weight, of the total composition, and in the case of using as an external preparation, the amount is usually 0.001 to 10% by weight, and preferably 0.1 to 5% by weight, of the total composition. The method of administering the agent for prevention/therapy of the present invention is not particularly limited, and the preparation is administered by a method selected in accordance with various preparation forms, the age, gender and other conditions, severity of the disease of the patient. For example, in the case of a tablet, a pill, a solution, a suspension, an emulsion, a granule and a capsule, the preparation is orally administered. In the case of an injection, the preparation is intravenously administered individually or as a mixture with a conventional replacement fluid such as glucose, amino acid or the like, or alternatively, is individually administeredintramuscularly,intradermally,subcutaneously or intraperitoneally as necessary. In the case of a suppository, the preparation is administered intrarectally. A spray preparation is administered by spraying intraorally or intranasally.

The pharmaceutically acceptable carrier for the medicine of the present invention may be exemplified by an excipient, a diluent, a bulking agent, a disintegrant, a stabilizer, a preservative, a buffering agent, an emulsifier, a fragrance, a colorant, a sweetening agent, a thickening agent, a flavoring agent, a dissolution aid, or other additives, or the like. By using one or more of such carriers, a pharmaceutical composition in the form of a tablet, a pill, a powder, a granule, an injection, a solution, a capsule, a troche, an elixir, a suspension, an emulsion, a syrup, an inhalant, an external aerosol, an external preparation such as a liquid coating preparation, a lotion, a liniment, an ointment, a cream, a PAP preparation or the like can be prepared, but an external preparation is preferred.

In the case of using the compound of the present invention in an oral preparation, the dose may be appropriately selected in accordance with the weight, age, gender, symptoms of the patient, but in the case of an adult, the amount to be administered is usually 1 to 3000 mg, and preferably 2 to 300 mg per day. In addition, the dosage may be administered once a day, or in two or more divided portions a day.

In the case of using the compound of the present invention in an external preparation, an amount to cover the entire affected site can be applied once or several times a day, by coating on the affected site, or the like.

### EXAMPLES

Hereinafter, the present invention will be described in detail with reference to Examples, but the invention is not intended to be limited thereto.

### EXAMPLE 1 Effect of the compound of the present invention on the growth of normal human keratinocyte (newborn infant foreskin-derived epidermal keratinocyte)

Normal human keratinocytes (purchased from Kurabo Industries, Ltd.) were inoculated on a 48-well plate (Coster Corp.) at a concentration of 4×10⁴ cells/well/500 µL, and were cultured overnight at 37°C in the presence of 5% CO₂. A compound of the present invention or activated vitamin D3 (Calcitriol; Wako Pure Chemical Industries, Ltd.) was added to a final concentration of 0.01 to 10 µM, and the cells were cultured for 22 hours. H³-thymidine (Amersham plc.) was added, and the cells were incubated for another 2 hours. Subsequently, the cells were recovered after washing, and in a liquid scintillation counter (Packard Instrument Company, Inc.), the radioactivity taken up by the cells was measured. The results are presented in Table 1. The results are presented as a ratio of the radioactivity taken up by the cells upon drug addition, with respect to the radioactivity of the case with no drug addition (Control), which was taken as 100%. The IC₅₀ value, which is the concentration of the drug for inhibiting the uptake of radioactivity to 50% with respect to the control, was lower in the case of anyof the Compounds 1 to 8 than in the case of Calcitriol. For example, the IC₅₀ values in the case of compound 4 and in the case of Calcitriol are respectively 0.247 and 1.9 µM, and the compound 4 exhibited about 8 times as much efficacy as that of Calcitriol. In psoriasis, abnormally increased growth of epidermal cells is most characteristic, and potent inhibition of the growth of epidermal cells is useful for the therapy of psoriasis.

**[Table 1]**

| | Human keratinocyte growth inhibition IC₅₀ (µM) |
|---|---|
| Compound 1 | 0.066 |
| Compound 2 | 0.044 |
| Compound 3 | 0.049 |
| Compound 4 | 0.247 |
| Compound 5 | 0.18 |
| Compound 6 | 0.177 |
| Compound 7 | >0.3 |
| Compound 8 | 1.0 |
| Calcitriol | 1.9 |

### EXAMPLE 2 Inhibitory effect of the compound of the present invention on skin thickening in TPA-stimulated hairless mouse

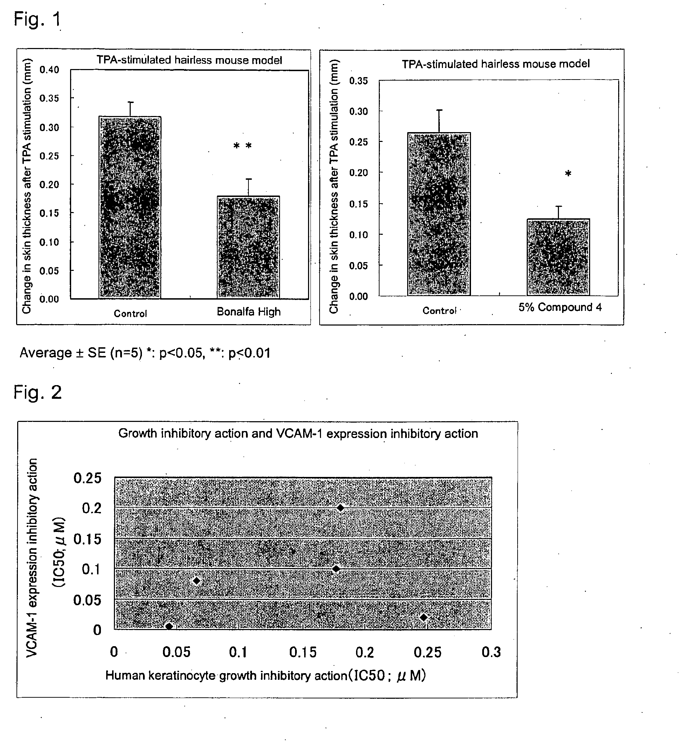

As an animal model for psoriasis, hairless mice stimulated with 12-O-tetradecanoylphorbol-13-acetate (hereinafter, referred to as TPA) were used (Sato, et al., Dermatology (1996) 192, 233-238). That is, on the dorsal side of a 7 to 8 weeks old female SKH1-hrBR-Hairless mouse (Charles River Laboratories Japan, Inc.), 10 nmol of TPA dissolved in acetone was applied, and then 50 µL of either the drug dissolved in acetone or Bonalfa (registered trademark) High (Teij in Pharma, Ltd.) was applied. After 24 hours, the thickness of the skin on the dorsal side was measured using a dial thickness gauge (Peacock G-1A, Ozaki MFG. Co., Ltd.), and the difference between the measured skin thickness and the skin thickness measured before TPA application was determined. The results are presented in Fig. 1. The thickening of the skin thickness was suppressed by the application of the activated vitamin D3 preparation (Bonalfa (registered trademark) High). Also, the thickening of the skin thickness was suppressed by the application of the Compound 4.

### EXAMPLE 3 VCAM-1 expression inhibitory activity

Human umbilical vein endothelial cells (HUVEC) (Sanko Junyaku Co. , Ltd.) were suspended in EGM-2 medium (Sanko Junyaku Co., Ltd.) at a concentration of 5×10⁴ cells/mL, and were inoculated on a 96-well plate (Corning Corp.) at a concentration of 1×10⁴ cells/well. The cells were cultured at 37°C in the presence of 5% CO₂ for 4 days, then 100 µL of the medium was removed, and 25 µL each of the substance under test and TNFα (final concentration 10 ng/mL, PeproTech, Inc.) were added. After incubating the cells for 4.5 hours at 37°C, the medium was removed, and each well was washed once with 100 µL of Hank's Balanced Salt Solution containing 0.5% bovine serum albumin and 0.02% sodium azide (HBSS/BSA). Subsequently, 30 µL of ablocking solution (1% whole goat serum (ICN Biomedicals, Inc.) /HBSS/BSA) was added, and the plate was left to stand for 30 minutes on the ice. After washing once with 100 µL of HBSS/BSA, 1 µg/mL of anti-human VCAM-1 antibody (Genzyme-Techne, Inc.) was added to each well in an amount of 30 µL, and the plate was incubated on the ice for 1 hour. After washing three times with HBSS/BSA (200 µL), peroxidase-labeled goat anti-mouse IgG antibody (Pierce Biotechnology, Inc.) diluted 2000-folds with HBSS/BSA was added in an amount of 30 µL/well, and was allowed to react for 1 hour. After washing 5 times with HBSS/BSA as described above, 100 µL/well of TMB substrate (Moss Inc.) was added, an enzymatic reaction was allowed to proceed for 15 to 30 minutes at 25°C, and then 1 mol/L sulfuric acid was added in an amount of 25 µL/well to stop the reaction. The absorbance at 492 nm was measured using a reference wavelength of 620 nm and using a96-wellplatespectrophotometer,Multiscan MS-UV (Labsystems, Inc.). The inhibitory rate was determined by taking the absorbance for the case of TNFα stimulation without drug addition, as 100%. In addition, the measurement was performed three times for each group. The results are presented in Table 2.

**[Table 2]**

| | VCAM-1 expression inhibitory action IC₅₀ (µM) |
|---|---|
| Compound 1 | 0.08 |
| Compound 2 | 0.005 |
| Compound 4 | 0.02 |
| Compound 5 | 0.2 |
| Compound 6 | 0.1 |
| Compound 7 | 0.2 |
| Compound 8 | 0.1 |

The results of comparing the inhibitory effect on the growth of cultured human epidermal cells and the VCAM-1 expression inhibitory action are presented in Fig. 2. There was no correlation between the inhibitory effect on the growth of cultured human epidermal cells and the VCAM-1 expression inhibitory action.

## Claims

1. An agent for the prevention/therapy of a disease caused by keratinocyte growth, the agent comprising, as an active ingredient, a compound represented by General Formula (1): 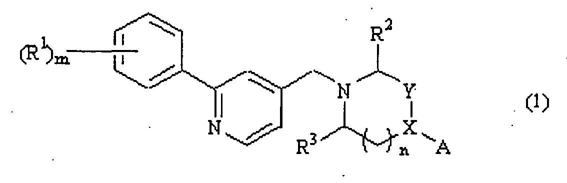 wherein A represents a group selected from the group consisting of the groups represented by the following formulas: 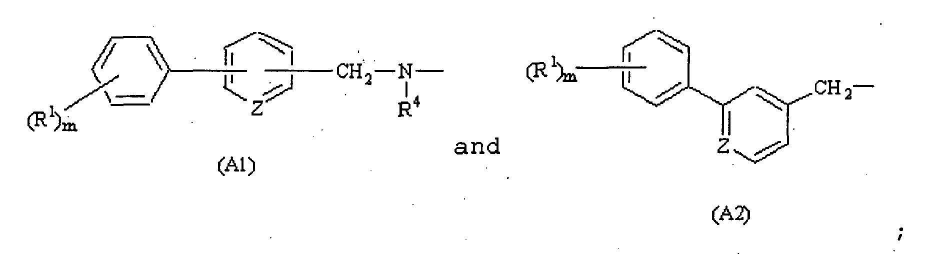 X represents CH or N;
Y represents CH₂ or C=O;
Z represents CH or N;
R¹ represents a C₁-C₆ straight-chained or branched alkoxy group;
R² and R³ each independently represent a C₁-C₆ straight-chained or branched alkyl group;
R⁴ represents a group selected from the group consisting of C₁-C₆ straight-chained or branched alkyl groups, and a group of the following formula: 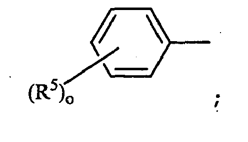 R⁵ each independently represent a C₁-C₆ straight-chained or branched alkoxy group or a C₁-C₆ straight-chained or branched alkylthio group;
m represents an integer from 1 to 3;
n represents an integer of 1 or 2; and
o represents an integer of 1 or 2,
or a salt thereof, or a solvate of the compound or the salt.

2. The agent for prevention/therapy according to claim 1,
wherein the active ingredient is a compound represented by Formula (1a): 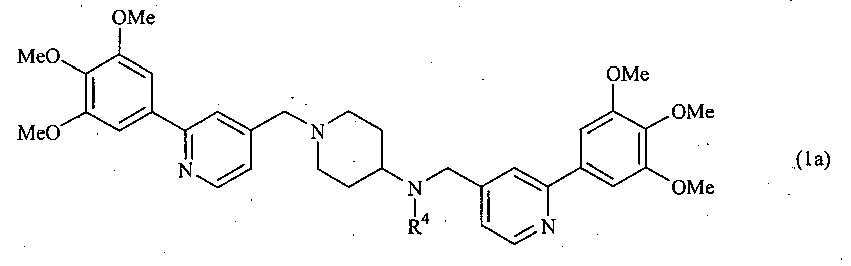 wherein R⁴ has the same meaning as defined above,
or a salt thereof, or a solvate of the compound or the salt.

3. The agent for prevention/therapy according to claim 1,
wherein the active ingredient is a compound selected from the group consisting of the compounds represented by the following formulas: 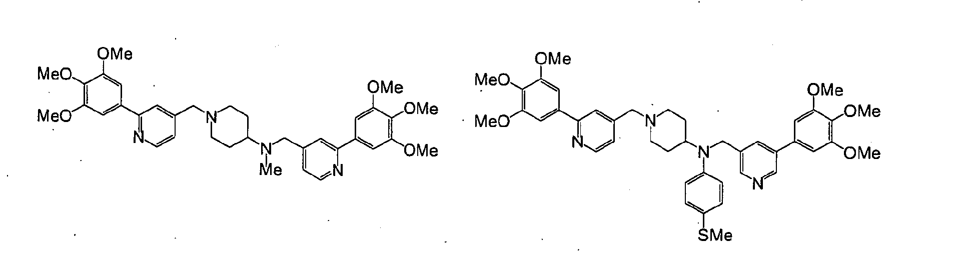 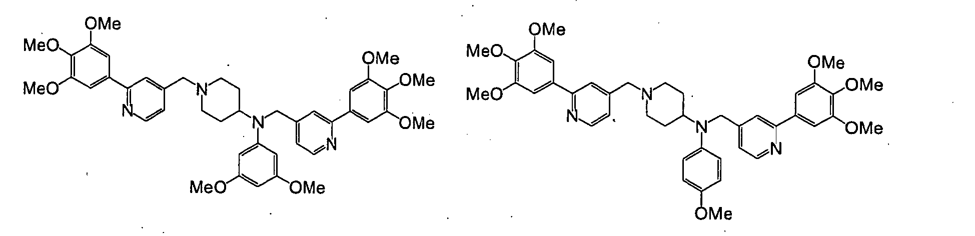 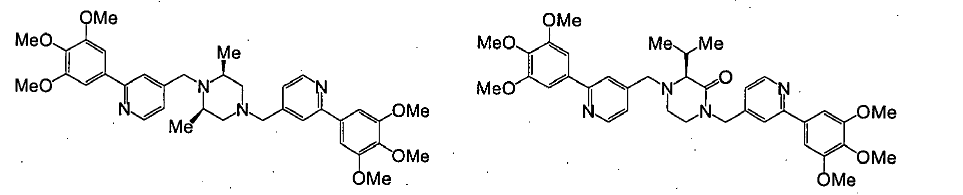 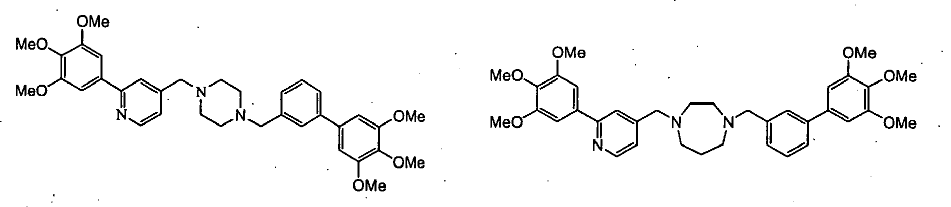 or a salt thereof, or a solvate of the compound or the salt.

4. The agent for prevention/therapy according to claim 1,
wherein the active ingredient is a compound selected from the group consisting of the compounds represented by the following formulas: 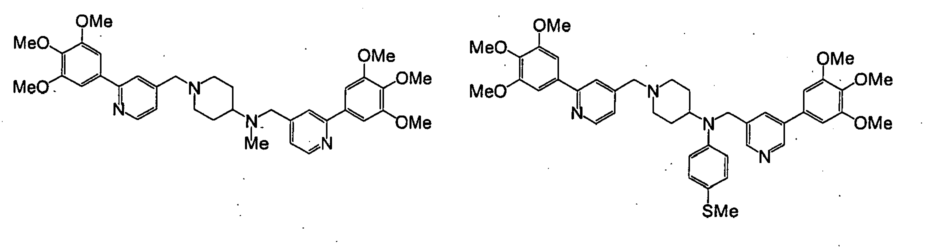 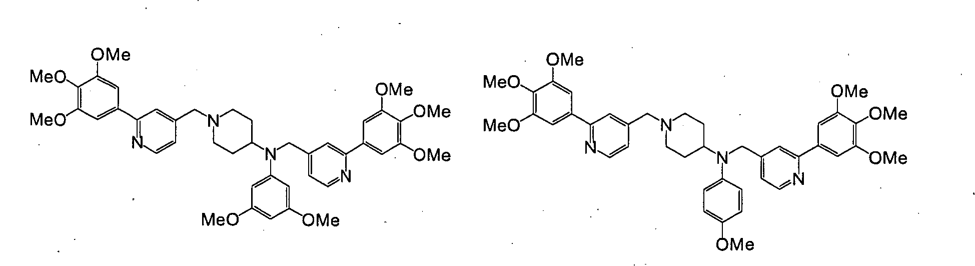 or a salt thereof, or a solvate of the compound or the salt.

5. The agent for prevention/therapy according to any one of claims 1 to 4,
wherein the disease is psoriasis.

6. The agent for prevention/therapy according to claim 5,
wherein the psoriasis is psoriasis vulgaris, psoriatic erythrodermia, arthropathic psoriasis, guttate psoriasis, or pustular psoriasis.

7. A method for the prevention/therapy of a disease caused by keratinocyte growth, the method comprising administering an effective amount of a compound represented by General Formula (1) : 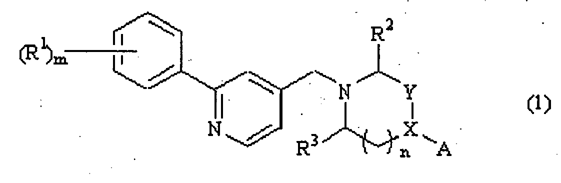 wherein A represents a group selected from the group consisting of the groups represented by the following formulas: 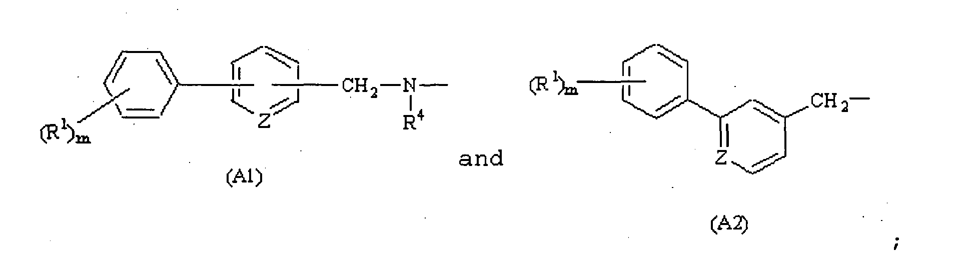 X represents CH or N;
Y represents CH₂ or C=O;
Z represents CH or N;
R¹ represents a C₁-C₆ straight-chained or branched alkoxy group;
R² and R³ each independently represent a C₁-C₆
straight-chained or branched alkyl group;
R⁴ represents a group selected from the groups consisting of C₁-C₆ straight-chained or branched alkyl groups, and a group of the following formula: 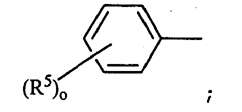 R⁵ each independently represent a C₁-C₆ straight-chained or branched alkoxy group or a C₁-C₆ straight-chained or branched alkylthio group;
m represents an integer from 1 to 3;
n represents an integer of 1 or 2; and
o represents an integer of 1 or 2,
or a salt thereof, or a solvate of the compound or the salt.

8. The method for prevention/therapy according to claim 7,
wherein an effective amount of a compound represented by Formula (1a) : 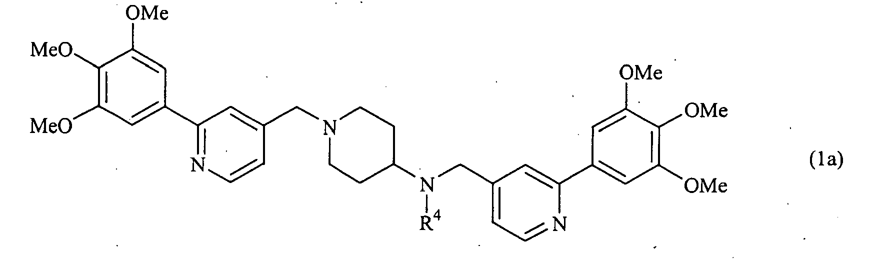 wherein R⁴ has the same meaning as defined above,
or a salt thereof, or a solvate of the compound or the salt is administered.

9. The method for prevention/therapy according to claim 7,
wherein an effective amount of a compound selected from the group consisting of the compounds represented by the following formulas: 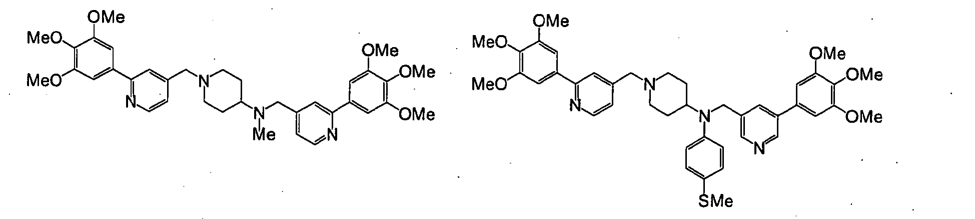 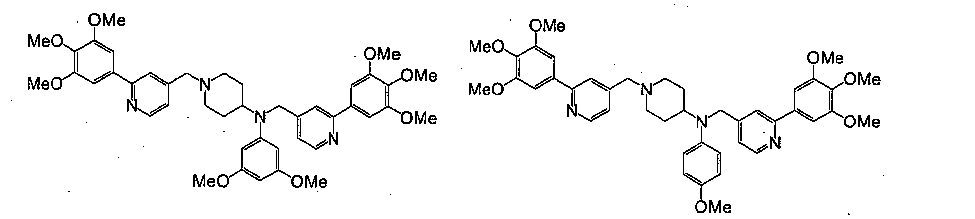 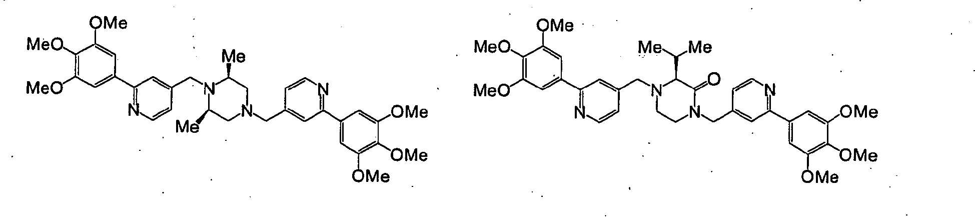 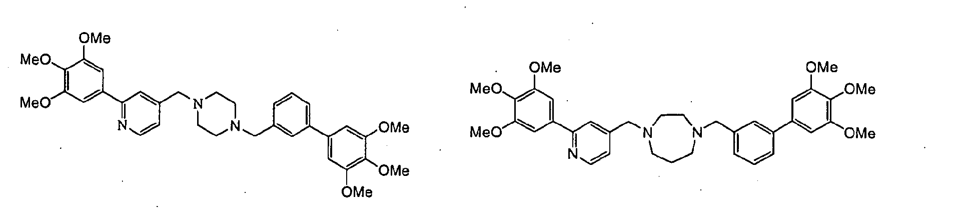 or a salt thereof, or a solvate of the compound or the salt is administered.

10. The method for prevention/therapy according to claim 7,
wherein an effective amount of a compound selected from the group consisting of the compounds represented by the following formulas: 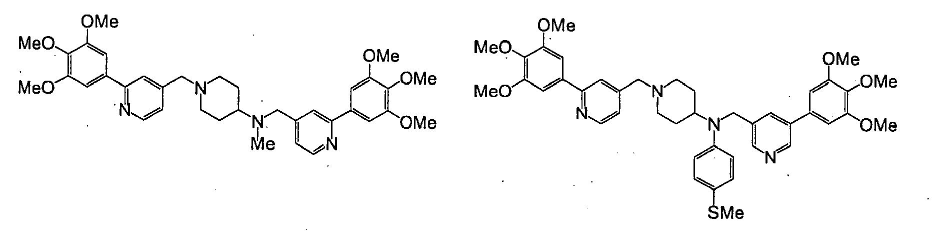 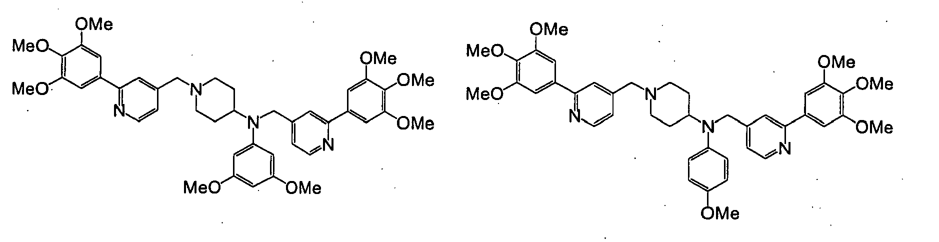 or a salt thereof, or a solvate of the compound or the salt is administered.

11. The method for prevention/therapy according to any one of claims 7 to 10,
wherein the disease is psoriasis.

12. The method for prevention/therapy according to claim 11,
wherein the psoriasis is psoriasis vulgaris, psoriatic erythrodermia, arthropathic psoriasis, guttate psoriasis, or pustular psoriasis.

13. Use of a compound represented by General Formula (1): 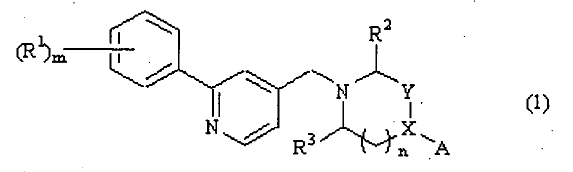 wherein A represents a group selected from the group consisting of the groups represented by the following formulas: 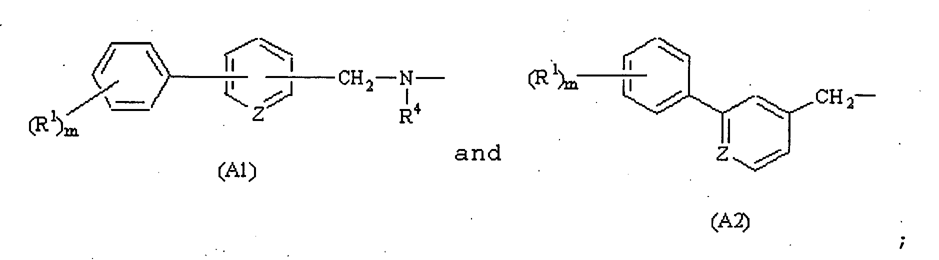 X represents CH or N;
Y represents CH₂ or C=O;
Z represents CH or N;
R¹ represents a C₁-C₆ straight-chained or branched alkoxy group;
R² and R³ each independently represent a C₁-C₆
straight-chained or branched alkyl group;
R⁴ represents a group selected from the group consisting of C₁-C₆ straight-chained or branched alkyl groups, and a group of the following formula: 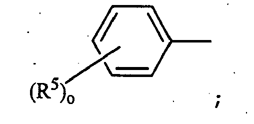 R⁵ each independently represent a C₁-C₆ straight-chained or branched alkoxy group or a C₁-C₆ straight-chained or branched alkylthio group;
m represents an integer from 1 to 3;
n represents an integer of 1 or 2; and
o represents an integer of 1 or 2,
or a salt thereof, or a solvate of the compound or the salt, for the manufacture of an agent for the prevention/therapy of a disease caused by keratinocyte growth.

14. The use according to claim 13,
wherein the compound represented by General Formula (1), a salt thereof, or a solvate of the compound or the salt is a compound represented by Formula (1a): 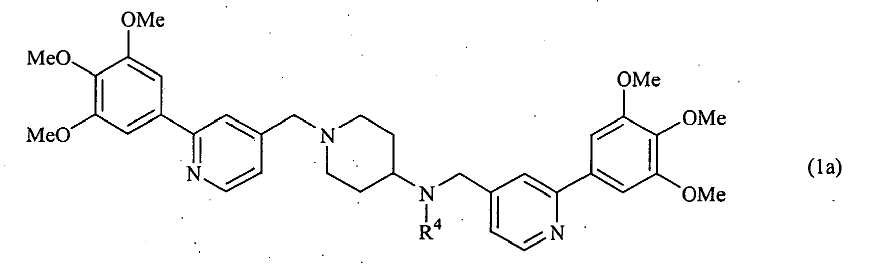 wherein R⁴ has the same meaning as defined above, or a salt thereof, or a solvate of the compound or the salt.

15. The use according to claim 13,
wherein the compound represented by General Formula (1), a salt thereof, or a solvate of the compound or the salt is a compound selected from the group consisting of the compounds represented by the following formulas: 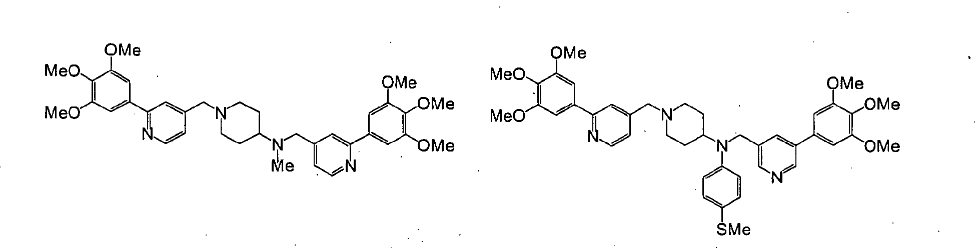 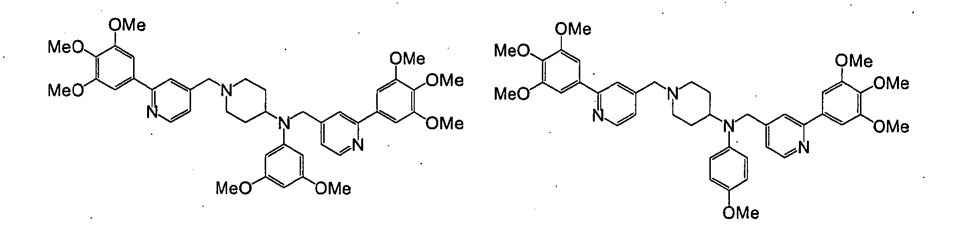 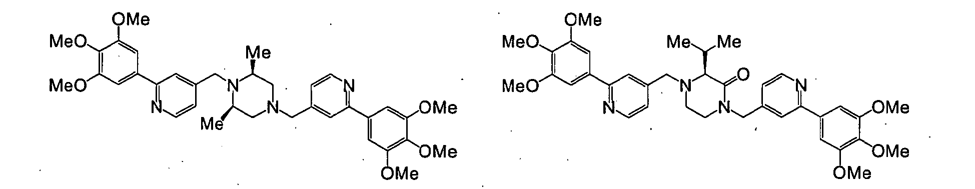 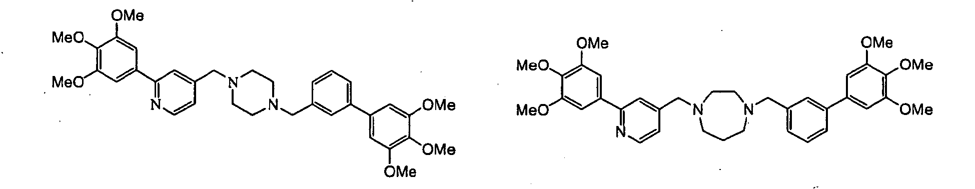 or a salt thereof, or a solvate of the compound or the salt.

16. The use according to claim 13,
wherein the compound represented by General Formula (1), a salt thereof, or a solvate of the compound or the salt is a compound selected from the group consisting of the compounds represented by the following formulas: 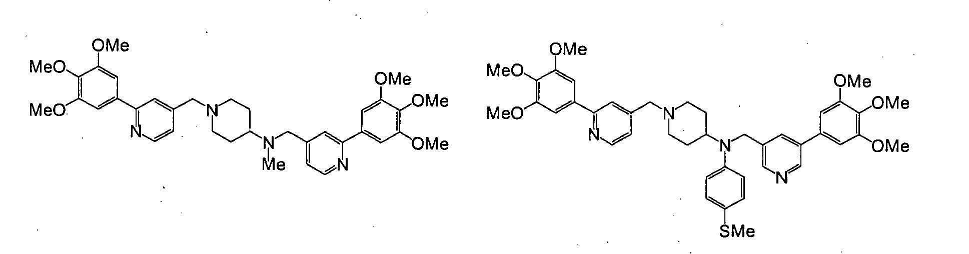 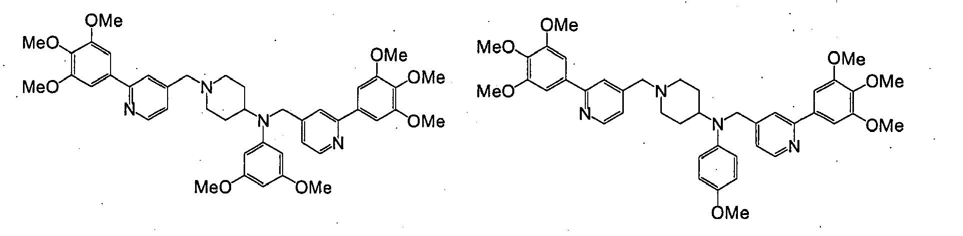 or a salt thereof, or a solvate of the compound or the salt.

17. The use according to any one of claims 13 to 16,
wherein the disease is psoriasis.

18. The use according to claim 17,
wherein the psoriasis is psoriasis vulgaris, psoriatic erythrodermia, arthropathic psoriasis, guttate psoriasis, or pustular psoriasis.
